# EUROPEAN PATENT SPECIFICATION

(11) **EP 2 468 745 B1**
(45) Date of publication and mention of the grant of the patent: **24.04.2013**
(21) Application number: 12158120.1
(22) Date of filing: 05.03.2012
(51) Int. Cl.: C07D 405/04, C07D 405/14, A61K 31/517, A61P 35/00

(54) **Process for the preparation of lapatinib and the salts thereof**
VERFAHREN ZUR HERSTELLUNG VON LAPATINIB UND DESSEN SALZE
PROCEDE DE PREPARATION DE LAPATINIB ET DE SES SELS

(30) Priority: 25.03.2011 IT MI20110480
(43) Date of publication of application: 27.06.2012
(73) Proprietor: F.I.S.- Fabbrica Italiana Sintetici S.p.A., 36075 Alte di Montecchio Maggiore (VI) (IT)
(72) Inventor: Fontana, Francesco, I-36075 Alte di Montecchio Maggiore, Vicenza (IT)
(74) Representative: Long, Giorgio

(56) References cited:
- US-A1- 2003 220 354

## Description

### Technical field of the invention

An object of the present invention is that of providing a process for the synthesis of the pharmaceutical active ingredient Lapatinib and the salts thereof.

### State of the art

Lapatinib is a pharmaceutical active ingredient used for the treatment of advanced metastatic lung cancer and it is currently available in the market under the name Tykerb® sold by GlaxoSmithKline (GSK).

According to the indications of the manufacturer, Tykerb® contains Lapatinib as monohydrate ditosylate salt of formula (I-bis): 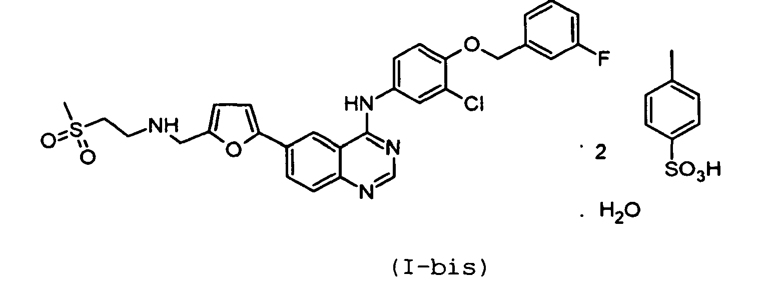 having the chemical name of N-{3-chloro-4-[(3-fluorobenzyl)oxy]phenyl}-6-[5-({[2-(methylsulfonyl)ethyl] amino}methyl)furan-2-yl]quinazoline-4-amine bis(4-methylbenzenesulfonate) monohydrate, CAS RN 388082-78-8 and m.p. 250-256°C.

This substance can be prepared following the teachings of the prior art such as for example those contained in US 7,157,466. In particular, in such reference, examples 10 and 11 show the preparation of the monohydrate ditosylate salt starting from anhydrous ditosylate salt.

The process above suffers from the main disadvantage consisting in some intermediates such as for example that having the aldehyde function in free form being poorly soluble thus leading to low productivity. In other processes of the known art stannane intermediates are used which entail problems related to the disposal of waste water.

### Summary of the invention

Thus, the problem addressed by the present invention regards providing a different process for the preparation of Lapatinib and the salts thereof, through a series of new intermediates capable of allowing at least partly overcoming the drawbacks revealed above with reference to the prior art.

This problem is resolved through a process for the synthesis of Lapatinib as outlined in the attached claims, whose definitions are an integral part of the present description.

Further characteristics and advantages of the process according to the invention will be apparent from the description - indicated hereinafter - of preferred embodiments, provided by way of non-limiting example.

### Brief description of the figures

By way of example:

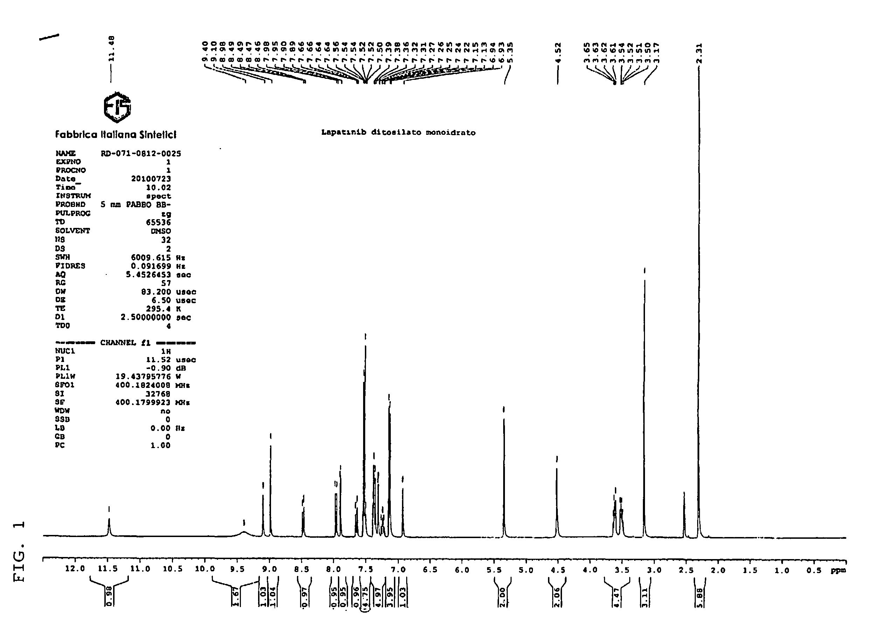

Figure 1 shows the 1H-NMR spectrum of Lapatinib ditosylate monohydrate obtained according to the process of the present invention.

### Detailed description of the invention

The present invention regards a process for the preparation of Lapatinib of formula (I) or a salt thereof: 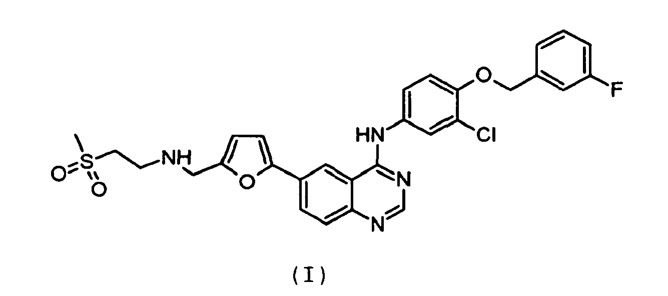 comprising the following steps:
(a) Reaction of 6-iodoquinazoline-4-ol of formula (II): 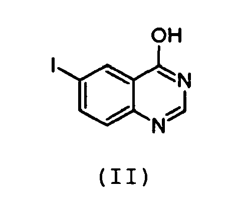 with 3,4-dihydro-2*H*-pyran to provide 6-iodo-4-(tetrahydro-2*H*-pyran-2-yloxy)quinazoline of formula (III): 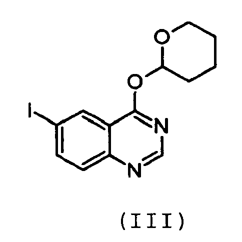
(b) Reaction of the 6-iodo-4-(tetrahydro-2*H*-pyran-2-yloxy)quinazoline of formula (III) with 2-formylfuran-5-boronic acid to provide 5-[4-(tetrahydro-2*H*-pyran-2-yloxy)quinazoline-6-yl]furan-2-carbaldehyde of formula (IV): 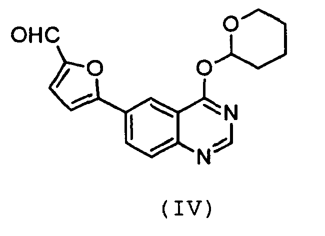
(c) Reaction of the intermediate of formula (IV) with 2-(methylsulfonyl)ethanamine to provide 2-(methylsulfonyl)-*N*-({5-[4-(tetrahydro-2*H-*pyran-2-yloxy)quinazoline-6-yl]furan-2-yl}methyl) ethanamine of formula (V): 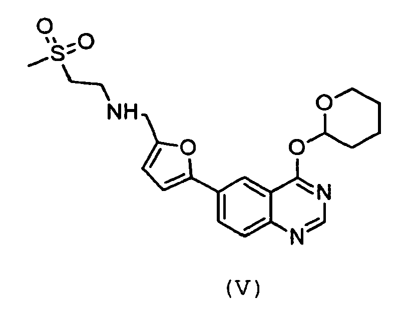
(d) Reaction of deprotection of the intermediate of formula ( V ) to provide 6-[5-({[2-(methylsulfonyl)ethyl]amino}methyl)furan-2-yl] quinazoline-4-ol of formula (VI): 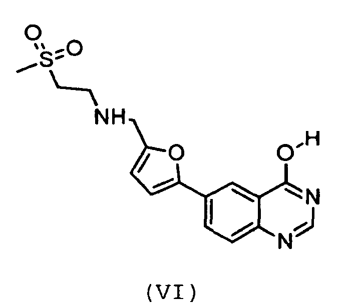
(e) Conversion of the intermediate of formula (VI) to provide *N*-{[5-(4-substituted-quinazoline-6-yl)furan-2-yl]methyl}-2-(methylsulfonyl) ethanamine of formula (VII): 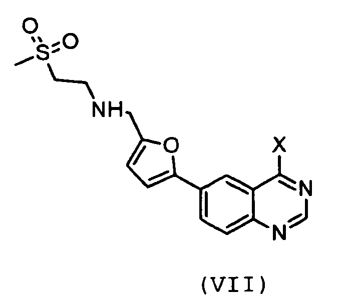 wherein X is selected from the group comprised of fluorine, chlorine, bromine, iodine, O-Ms (mesylate), O-Ts (tosylate), O-Tf (triflate);
(f) Reaction of the intermediate of formula (VII) with the 3-chloro-4-[(3-fluorobenzyl)oxy]aniline of formula (VIII): 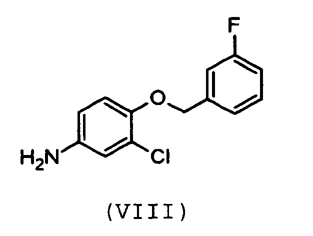 to provide Lapatinib of formula (I).
(g) Optionally, conversion of the Lapatinib of formula (I) into Lapatinib ditosylate monohydrate.

In an embodiment, the step (a) of preparation of the 6-iodo-4-(tetrahydro-2H-pyran-2-yloxy)quinazoline intermediate of formula (III): 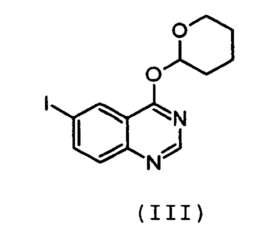 using tetrahydropyranyl (THP) group starting from 6-iodoquinazoline-4-ol of formula (II) may be conveniently performed in an organic solvent such as for example Toluene or mixtures such as for example AcOEt/DMF (8:2); preferably the reaction is performed in toluene. Trifluoroacetic acid in a catalyst amount can be used as a catalyst to facilitate the reaction. The reaction can be performed at T=110-115°C (at reflux) for 2-3 hours. The conversion reaction is free of by-products and it is almost quantitative. The raw product may optionally be purified by means of recrystalisation from 5 volumes of AcOEt, for an overall molar yield of about 73%.

An advantage of this process lies in the fact that the 6-iodo-4-(tetrahydro-2H-pyran-2-yloxy)quinazoline of formula (III) is more soluble with respect to the initial product hence allowing always operating in homogeneous phase and performing possible recrystallizations using small volumes of solvent. Furthermore, the commercial cost of 3,4-dihydro-2*H*-pyran (DHP) is very low.

In an embodiment, the step (b) of preparation of the 5-[4-(tetrahydro-2*H*-pyran-2-yloxy)quinazoline-6-yl]furan-2-carbaldehyde intermediate of formula (IV): 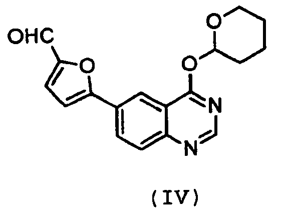 through a cross-coupling reaction of the 6-iodo-4-(tetrahydro-2*H*-pyran-2-yloxy)quinazoline intermediate of formula (III) with 2-formylfuran-5-boronic acid (CAS RN 27329-70-0) is performed in an organic solvent, such as dimethylformamide in presence of palladium tris dibenzylideneacetone and triphenylarsine catalyst system. Other ligands such as triphenylphosphine can be used alternatively. The reaction is performed in the presence of a base, such as for example potassium carbonate. The reaction is necessarily performed in anhydrous conditions and in absence of oxygen.

This reaction proceeds with molar yields ranging between 70% and 90%. The product of formula (IV) can be optionally purified by means of pulping from ethyl acetate for an overall molar yield of about 83%. An advantage lies in the fact that the use of 2-formylfuran-5-boronic acid allows avoiding the use of stannanes which are typically used for adding the 2-formylfuran group according to the processes of the prior art regarding this type of coupling, with a clear advantage in terms of toxicity and disposal. 2-formylfuran-5-boronic acid is a substance available in the market.

In an embodiment, the step (c) of preparation of the 2-(methylsulfonyl)-*N*-({5-[4-(tetrahydro-2*H*-pyran-2-yloxy)quinazoline-6-yl]furan-2-yl}methyl)ethanamine intermediate of formula (V): 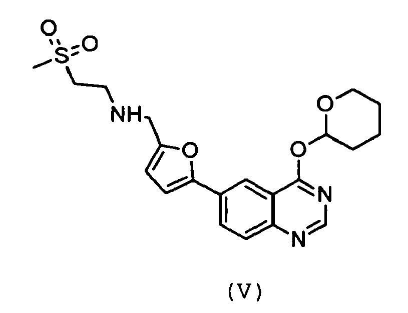 through the reductive amination reaction of 5-[4-(tetrahydro-2*H*-pyran-2-yloxy)quinazoline-6-yl]furan-2-carbaldehyde intermediate of formula (IV) with 2-(methylsulfonyl)ethanamine, is performed in an organic solvent, preferably in dichloromethane and in presence of sodium triacetoxyborohydride and Diisopropylethylamine (DIPEA). 2-(methylsulfonyl)ethanamine as a hydrochloride is a product available in the market (CAS RN 104458-24-4).

The 2-(methylsulfonyl)-*N*-({5-[4-(tetrahydro-2*H*-pyran-2-yloxy)quinazoline-6-yl]furan-2-yl}methyl) ethanamine intermediate of formula (V) is deemed a key intermediate of the entire process in that the product can be obtained therefrom also with variants to the present process. For example, such intermediate can be directly converted into the *N*-{[5-(4-bromoquinazoline-6-yl)furan-2-yl]methyl}-2-(methylsulfonyl)ethanamine intermediate of formula (VII, X=Br): 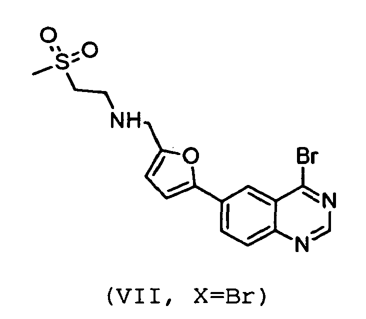 or into the *N*-{[5-(4-iodoquinazoline-6-yl)furan-2-yl]methyl}-2-(methylsulfonyl)ethanamine intermediate of formula (VII, X=I): 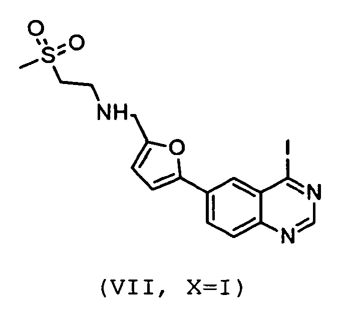 using the conditions (LiBr or NaI and BF3.Et2O or trimethylchlorosilane) described by Yashwant D. Vankar et al. in Tetrahedron Letters, vol. 32, n.8, 1081-1084 (1991) for the direct conversion of tetrahydropyranyl ethers to the corresponding bromides and iodides. Such intermediates of formula (VII, X=Br) and (VII-X=I) are subjected to the same nucleophilic substitution reaction of the corresponding chlorine derivative of formula (VII) or of the corresponding mesylates of formula (VII) to provide Lapatinib according to step (f).

In an embodiment, the step (d) of preparation of the 6-[5-({[2-(methylsulfonyl)ethyl]amino}methyl)furan-2-yl] quinazoline-4-ol intermediate of formula (VI): 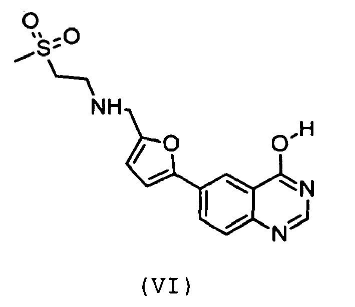 through the deprotection of the 2-(methylsulfonyl)-N-({5-[4-(tetrahydro-2*H*-pyran-2-yloxy)quinazoline-6-yl]furan-2-yl}methyl)ethanamine intermediate of formula (V) may be conveniently performed in alcohol, for example in methanol, in presence of an acid catalyst such as for example methanesulfonic acid or paratoluenesulfonic acid. The reaction is performed at ambient temperature for at least 1 hour obtaining molar yields in the order of 95%.

In an embodiment, the step (e) of preparation of *N*-{[5-(4-substituted-quinazoline-6-yl)furan-2-yl]methyl}-2-(methylsulfonyl)ethanamine of formula (VII) 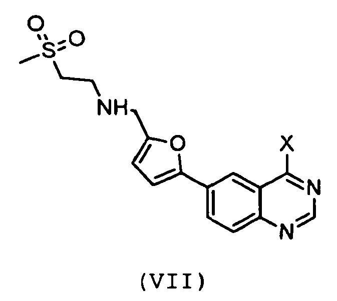 is performed in an organic solvent, typically in toluene, by means of halogenation reagents such as for example POCl₃, SO₂Cl, (COCl)₂ and PBr₃ or by means of sulfonyl halogenides and sulfonic anhydrides such as for example mesyl chloride, tosyl chloride, tosyl anhydride and triflic anhydride.

In a preferred embodiment, the step (e) of preparation of the *N*-{[5-(4-chloroquinazoline-6-yl)furan-2-yl]methyl}-2-(methylsulfonyl)ethanamine intermediate of formula (VII, X=Cl): 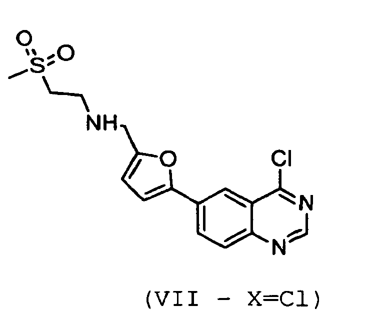 through the halogenation of the 6-[5-({[2-(methylsulfonyl)ethyl]amino}methyl)furan-2-yl] quinazoline-4-ol intermediate of formula (VI) can be performed in an organic solvent such as for example toluene by means of phosphoryl oxychloride (POCl₃) in presence of a base such as for example triethylamine (TEA). Other chloride agents such as thionyl chloride or oxalyl chloride can be used alternatively, phosphorus oxychloride being preferred.

In an embodiment, the step (f) of preparation of the Lapatinib of formula (I): 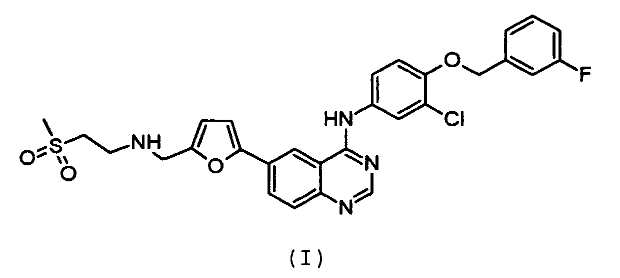 through the coupling reaction of the *N*-{[5-(4-substituted-quinazoline-6-yl)furan-2-yl]methyl}-2-(methylsulfonyl)ethanamine intermediate of formula (VII) with the 3-chloro-4-[(3-fluorobenzyl)oxy]aniline of formula (VIII): 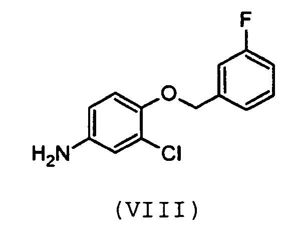 may be conveniently performed in an alcoholic solvent, preferably in isopropanol and at a temperature comprised between 60°C and 75°C. Should the compound of formula VII have X=Cl, the product can be conveniently isolated directly as a hydrochloric salt, while should X be OTs, the product could be conveniently isolated directly as Lapatinib monotosylate.

In an embodiment, the optional step (g) for preparing Lapatinib ditosylate monohydrate can be performed by first obtaining Lapatinib ditosylate thus subjecting such compound to hydration reaction in water. These methods for preparing Lapatinib ditosylate monohydrate are known in literature.

The intermediate compounds of the present invention having the tetrahydropyranyl group are extremely soluble in organic solvents hence making them particularly interesting for industrial application in that they allow operating in much more concentrated solutions with respect to the ones used in the current industrial synthesis of the Lapatinib according to the description of US 7,157,466. This allows producing with a much larger product batch size. The process of the present invention thus reveals high productivity thus leading to a considerable reduction of the Lapatinib industrial production costs.

### EXPERIMENTAL PART

Example 1 - preparation of the intermediate 6-iodo-4-(tetrahydro-2*H*-pyran-2-yloxy)quinazoline of formula (III) - exemplifying the invention

### Synthesis scheme

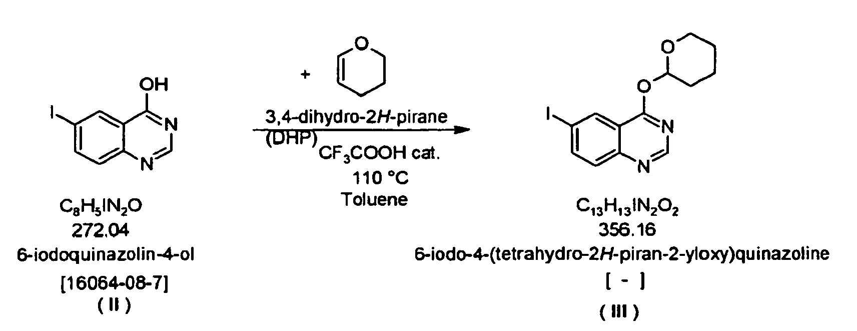

In a 4-neck glass flask provided with a mechanical stirrer, condenser and thermometer 30 g of 6-iodoquinazoline-4-ol (110 mmoles), 300 mL of toluene dried on molecular sieves, 1.26 g (0.84 mL) of trifluoroacetic acid (0.1 mol. equiv.) and 79.4 g (80 mL) of 3,4-dihydro-2*H*-pyran (DHP) (944 mmoles; 8.6 mol. equiv.) were introduced, under nitrogen. Stirring is carried out to reflux (T=110-115°C) for 2-3 hours and the reaction is controlled under TLC with Hexane/Ethyl Acetate (2:8) eluent. Upon completing the reaction a homogeneous solution is obtained which is cooled to ambient temperature and washed using 2x120 mL of saturated solution of sodium chloride. The organic phase is concentrated under vacuum to 40-45°C ext T up to residue.

The product is recrystalised by adding 150 mL of Ethyl acetate and heating to reflux. Cooling is carried out up to ambient temperature and stirring is carried out for 30 minutes then cooling is carried out at 0-5°C and stirring is carried out for 30 minutes. The suspension is filtered and the solid is washed with 60 mL of ethyl acetate pre-cooled at 0-5°C. The product is dried under vacuum at 40°C for 6-8 hours obtaining 28.6 g of the product as a white crystalline solid for a molar yield equivalent to 72.8%.

1H-NMR (400 MHz, DMSO-d6): 1.75 (m, 6H, CH2(THP)); 3.69 (dt, J = 11.6, 2.9 Hz, 1H, CH2O(THP)); 4.11 (app. d, J = 11.6 Hz, 1 H, CH2O(THP)); 5.85 (dd, J = 8.7, 4.2 Hz, 1H, OCHO(THP)); 7.50 (d, J = 8.5 Hz, 1 H, H-8); 8.16 (dd, J = 8.5, 1.5 Hz, 1H,H-7); 8.45 (d, J = 1.8 Hz, 1H, H-5); 8.45 (s, 1H, H-2).

Example 2 - Preparation of the 5-[4-(tetrahydro-2*H-*pyran-2-yloxy)quinazoline-6-yl]furan-2-carbaldehyde intermediate of formula (IV) - exemplifying the invention

### Synthesis scheme

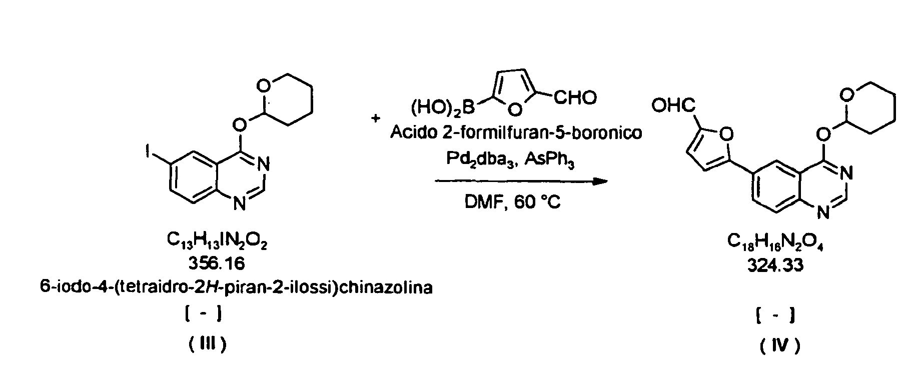

Acido 2-formilfuran-5-boronico = 2-formylfuran-5-boronic acid
6-iodo-4-(tetraedro-2H-piran-2-ilossi)chinazolina = 6-Iodo-4-(tetrahydro-2H-pyran-2-yloxy)quinazoline

In a 4-neck glass flask provided with a mechanical stirrer, condenser and thermometer, the entirety having been previously dried, 320 mg of palladium tris dibenzylideneacetone (Johnson-Mattey - Pd-94; 1.25% mol.) weighed under nitrogen and 430 mg of triphenylarsine (Aldrich) (0.025 mol. equiv.) were introduced, under nitrogen atmosphere. 200 mL of previously degassed anhydrous DMF are added under nitrogen for 1 hour. Stirring is carried out for 10-15 minutes at ambient temperature then there are added 15.5 g of potassium carbonate (2 mol. equiv.) and 10.2 g of 2-formylfuran-5-boronic acid (1.3 mol. equiv.) and lastly 20.0 g of 6-iodo-4-(tetrahydro-2*H*-pyran-2-yloxy)quinazoline of formula (III). The reaction mixture is heated for 2 hours at 60-65°C. The reaction may be controlled by means of TLC using Hexane/AcOEt (6:4) as eluent.

Upon completing the reaction there are added 200 mL of purified water and extraction is carried out with 2x500 mL of dichloromethane. The phases are separated and the aqueous phase is washed with 2x300 mL of NaHCO₃ at 5%, then with 2x300 mL of a saturated solution of sodium chloride. The organic phase is then dried with anhydrous sodium sulfate then with 2.0 g of Acticarbone and filtered on a dicalite panel which is then washed with 2x100 mL of dichloromethane. The solution is washed, concentrated to residue under vacuum at 35-40°C ext T.

The residue, a yellow/orange solid, is recovered using 200 mL of AcOEt.

The stirring is carried out at 20-25°C for 30 minutes and then cooling is carried out at 0-5°C and stirring is carried out for another 30 minutes. The suspension is filtered and the solid washed with 80 mL of AcOEt pre-cooled at 0-5°C. The solid is dried in an oven at 35-40°C for 4-5 hours. There are obtained 13.5 g of product for a molar yield equivalent to 74.1%. 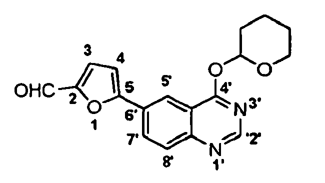 1H NMR (400 MHz, DMSO-d6): 1.77 (m, 6H, CH2(THP)); 3.73 (dt, J = 11.6, 2.7 Hz, 1H, CH2O(THP)); 4.13 (app. dd, J = 11.0, 1.6 Hz, 1 H, CH2O(THP)); 5.90 (dd, J = 8.2, 4.6 Hz, 1H, OCHO(THP)); 7.53 (d, J = 3.7 Hz, 1 H, CH(furan)); 7.72 (d, J = 3.7 Hz, 1 H, CH(furan)); 7.84 (d, J = 8.6 Hz, 1H, H-8'); 8.48 (dd, J = 8.5, 1.9 Hz, 1H,H-7'); 8.51 (s, 1H, H-2'); 8.59 (d, J = 1.6 Hz, 1H, H-5'); 9.68 (s, 1H, CHO).

Example 3 - Preparation of the 2-(methylsulfonyl)-N-({5-[4-(tetrahydro-2*H*-pyran-2-yloxy)quinazoline-6-yl]furan-2-yl}methyl)ethanamine intermediate of formula (V)- exemplifying the invention

### Synthesis scheme

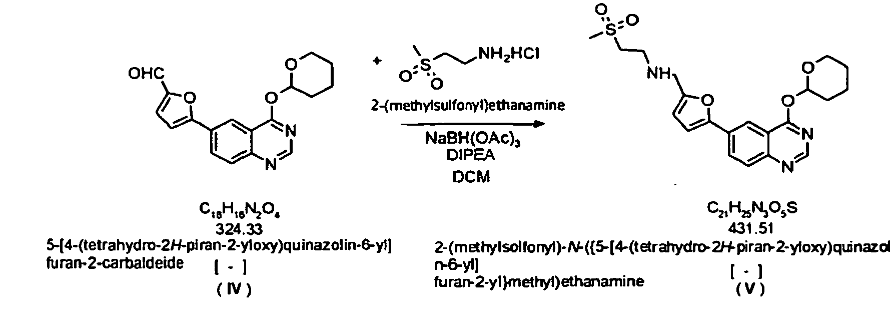

In a 4-neck glass flask provided with a mechanical stirrer, condenser and thermometer, the entirety having been previously dried, 1.0 g of 5-[4-(tetrahydro-2*H-*pyran-2-yloxy)quinazoline-6-yl]furan-2-carbaldehyde of formula (IV) and 20 mL of anhydrous dichloromethane were introduced, under nitrogen atmosphere. There are added, at 20-25°C, 0.73 g of 2-(methylsulfonyl)ethanamine hydrochloride (1.5 mol. equiv.) and 0.60 g (0.80 mL) of diisopropylethylamine (DIPEA) (1.5 mol. equiv.). Stirring is carried out for 30 minutes at 30-35°C. Cooling is carried out at 20-25°C and 1.31 g of triacetoxy sodium borohydride (Aldrich) (2.0 mol. equiv.) are added. Stirring is carried out at 20-25°C for 2 hours then the conversion is controlled under TLC using the Hexane/Ethyl acetate 6:4 mixture as eluent. Upon completing the reaction 10 mL of a saturated solution of sodium bicarbonate are added, the phases separated and the organic phase is dried on anhydrous sodium sulphate, whereupon it is concentrated under vacuum at 35-40°C ext T up to residue. The product is dried by means of high vacuum (using an Edwards pump). There are obtained 1.06 g of product for a molar yield equivalent to 80%.

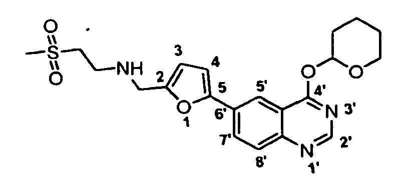

1H NMR (400 MHz, DMSO-d6): 1.86 (m, 6H, CH2(THP)); 2.99 (t, J = 6.6 Hz, 2 H, -SO2CH2CH2NH-); 3.05 (s, 3 H, CH3); 3.28 (t, J = 6.6 Hz, 2 H, -SO2CH2CH2NH-); 3.71 (dt, J = 11.8, 2.9 Hz, 1H, CH2O (THP)); 3.83 (s, 2 H, -NH-CH2-furan); 4.12 (app. d, J = 11.0, 1 H, CH2O(THP)); 5.90 (dd, J = 8.8, 4.4 Hz, 1H, OCHO(THP)); 6.46 (d, J = 3.2 Hz, 1 H, CH (furan)) ; 7.10 (d, J = 3.2 Hz, 1 H, CH(furan)); 7.74 (d, J = 8.6 Hz, 1H, H-8'); 8.17 (dd, J = 8.5, 2.0 Hz, 1H, H-7'); 8.38 (d, J = 1.8 Hz, 1H, H-5'); 8.43 (s, 1H, H-2').

Example 4 - Preparation of the intermediate 6-[5-({[2-(methylsulfonyl)ethyl]amino}methyl)furan-2-yl] quinazoline-4-ol of formula (VI) - exemplifying the invention

### Synthesis scheme

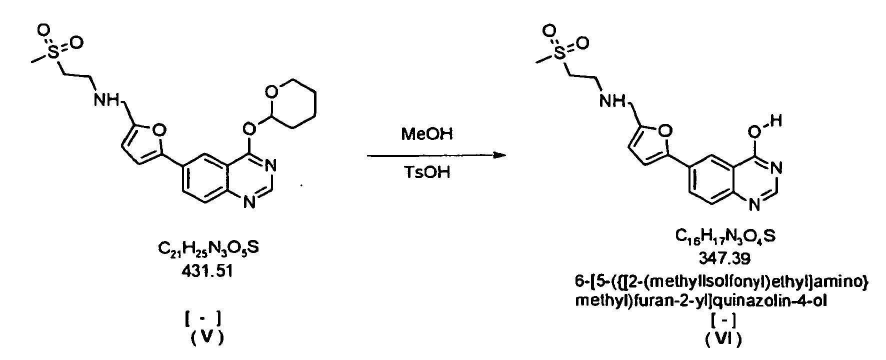

In a 4-neck glass flask provided with a mechanical stirrer, condenser and thermometer, the entirety having been previously dried, 1.0 g of 2-(methylsulfonyl)-N-({5-[4-(tetrahydro-2*H*-pyran-2-yloxy)quinazoline-6-yl]furan-2-yl}methyl)ethanamine of formula (V), 20 mL of methanol and 0.5 g of paratoluenesulfonic acid were introduced. The product is left under stirring at 25°C for at least 1 hour. Upon completing the reaction there are added 50 mL of AcOEt and 50 mL of 5% aqueous NaHCO₃. The phases are separated. The organic phase is concentrated to residue under low pressure and 35°C ext T.

There are obtained 0.76 g of product with a 95% molar yield.

Example 5 - Preparation of the intermediate *N*-{[5-(4-chloroquinazoline-6-yl)furan-2-yl]methyl}-2-(methylsulfonyl)ethanamine of formula (VII) - exemplifying the invention

### Synthesis scheme

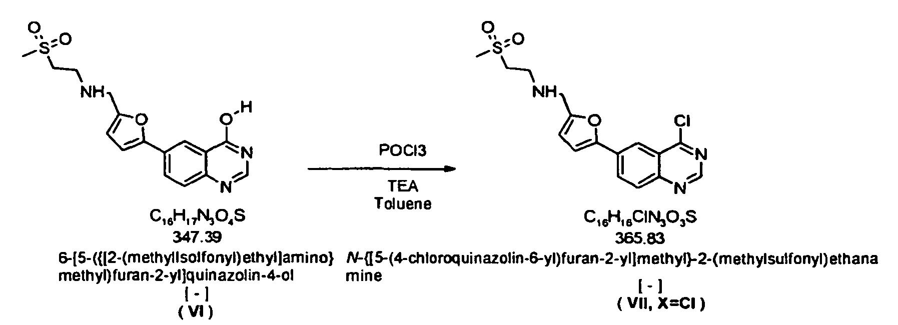

In a 4-neck glass flask provided with a mechanical stirrer, condenser and thermometer, the entirety having been previously dried, 63.8 g of 6-[5-({[2-(methylsulfonyl)ethyl]amino}methyl)furan-2-yl] quinazoline-4-ol of formula (VI)(0.184 moles), 33.7 g (20.1 mL) of phosphoryl oxychloride (POCl₃; 0.220 moles) and 150 mL of toluene were introduced, under nitrogen. Stirring is carried out at ambient temperature for 10 minutes then 22.3 g (30.7 mL) of triethylamine (0.220 moles) are dosed, maintaining the T below 30°C. After introduction heating is carried out at 75°C for 3 hours. The reaction is controlled by means of TLC using the Hexane/Ethyl acetate mixture (4:6) as eluent. Cooling is carried out at 0°C and stirring is carried out for an hour at such temperature. The suspension is filtered washing the solid with 100 mL of toluene. The product is dried at 50°C for 7-8 hours under vacuum.

There are obtained 63.8 g of product with a molar yield equivalent to 95%.

Example 6 - Preparation of Lapatinib of formula (I) as a hydrochloride salt:
- exemplifying the invention

### Synthesis scheme

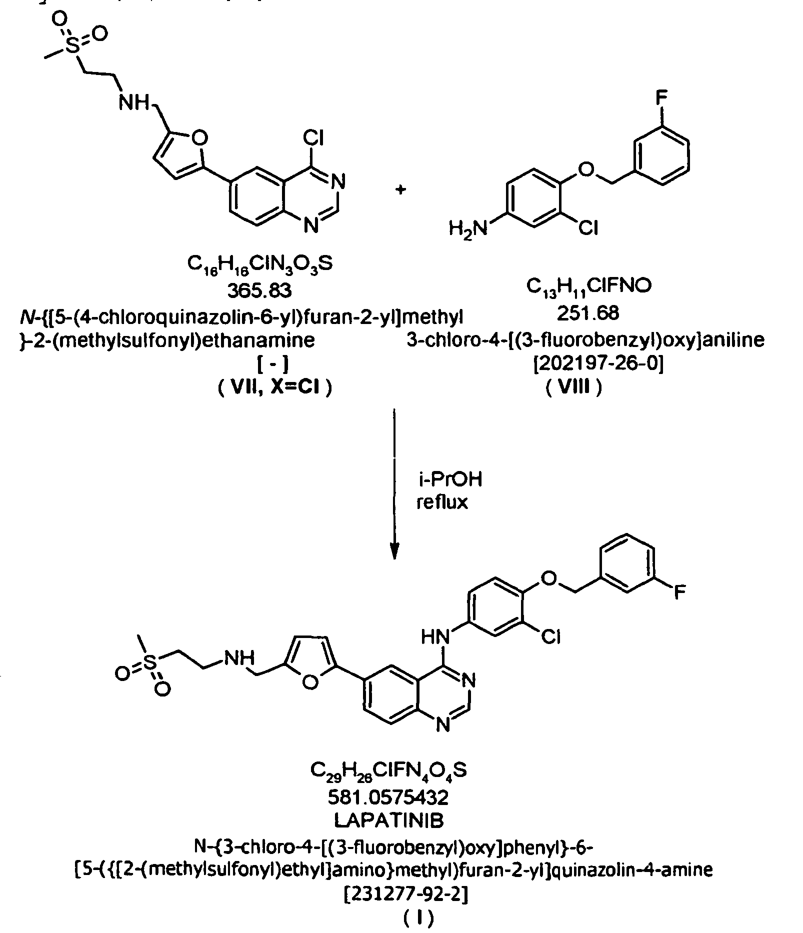

In a 4-neck glass flask provided with a mechanical stirrer, condenser and thermometer, the entirety having been previously dried, 10.0 g of 3-chloro-4-[(3-fluorobenzyl)oxy]aniline of formula (VIII) (39.7 mmoles), 14.6 g of *N*-{[5-(4-chloroquinazoline-6-yl)furan-2-yl]methyl}-2-(methylsulfonyl)ethanamine of formula (VII) (40.0 mmoles, 1.005 mol. equiv.) and 200 mL of isopropanol were introduced, under nitrogen. Stirring is carried out at 70°C for 4 hours. Upon completing the reaction, cooling is carried out at ambient temperature. It is left under stirring for 1 hour. The solid is filtered and washed with 14 mL of cold isopropanol.

It is dried under vacuum at 40°C for 4-6 hours. There are obtained 23.5 g of product with a molar yield equivalent to 96%.

Example 7 - Preparation of Lapatinib of formula (I) as a ditosylate salt - exemplifying the invention.

In a 4-neck glass flask provided with a mechanical stirrer, condenser and thermometer, 9.70 g of Lapatinib chlorohydrate (15.7 mmoles), 120 ml of ethyl acetate and 25 mL of purified water were introduced under nitrogen. About 4.6 g of NaOH 30% solution (p/p) are dripped up to a pH of about 10. The phases are separated and the organic phase is washed with 2x30 mL of purified water.

The organic phase is concentrated under vacuum to dryness.

The residue is recovered using 28 mL of dimethylformamide. Heating is carried out at 40°C for 15 minutes and filtration is carried out on a dicalite panel. The panel is washed with 21 ml of dimethylformamide pre-heated at 50°C. The organic phases are combined, they are brought to 40°C and they are added portionwise with 6.74 g of paratoluenesulfonic acid monohydrate (2.25 mol. equiv.). Stirring is carried out at 40°C for 1 hour and then cooling is carried out within 3-4 hours at 0°C. 0.08 g of Lapatinib ditosylate are added to initiate the precipitation and stirring is carried out for 4 hours at 0°C, then cooling is carried out at -10°C and stirring is carried out for 2 hours. The suspension is filtered and the solid is washed with 4.8 mL of dimethylformamide pre-cooled to -10°C. The product is dried under vacuum at 70°C for at least 10 hours. 13.1 g of product are obtained with a molar yield equivalent to 90.1%.

Example 8 - Preparation of Lapatinib ditosylate monohydrate formula (I-bis) - exemplifying the invention.

In a 4-neck glass flask provided with a mechanical stirrer, condenser and thermometer, 50.0 g of Lapatinib ditosylate and 500 mL of water were introduced under nitrogen. Stirring is carried out for 36 hours at ambient temperature. Filtration is carried out draining the product thoroughly and the product is washed using the mother liquors. The product is dried at ambient temperature under the flow of nitrogen in a flask provided with a stirrer.

The product is thus dried for 24 hours at 55°C up to K.F. around 1.92%. There are obtained 51 g of product for a quantitative molar yield.

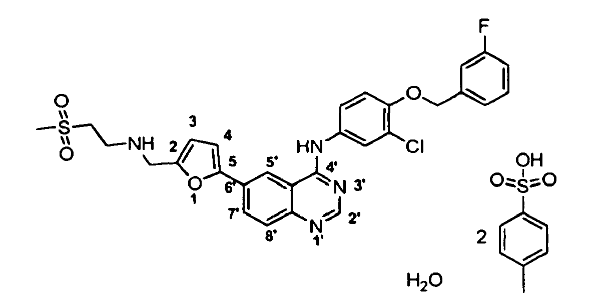

1H NMR (400 MHz, DMSO-d6): □2.31 (s, 6 H, CH3 (TsOH)); 3.17 (s, 3 H, CH3SO2); 3.50-3.65 (m, 4 H, -SO2CH2CH2NH-); 4.52 (s, 2, NH-CH2-furan); 5.35 (s, 2 H, ArO-CH2-Ar); 6.93 (d, J = 3.4 Hz, 1 H, CH (furan)) ; 7.14 (d, J = 7.8 Hz, 4 H, CH(TsOH)); 7.24 (dt, J = 8.8, 2.1 Hz, 1 H, Ar); 7.32 (d, J = 3. 4 Hz, 1 H, CH (furan)) ; 7.53 (d, J = 8.0 Hz, 4 H, CH(TsOH)); 7.65 (dd, J = 8.9, 2.5 Hz, Ar); 7.90 (d, J = 2.6 Hz, 1H, H-5'); 7.97 (d, J = 8.8 Hz, 1 H, H-8'); 8.48 (dd, J = 8.8, 1.5 Hz, 1 H, H-7'); 8.99 (s, 1 H, H-2'); 9.10 (s, 1 H, Ar); 9.40 (br. s, 1 H, NH); 11.48 (s, 1 H, NH).

Example 9 - Preparation of *N*-{[5-(4-bromoquinazoline-6-yl)furan-2-yl]methyl}-2-(methylsulfonyl)ethanamine of formula (VII, X=Br) (variant)- exemplifying the invention.

### Synthesis scheme

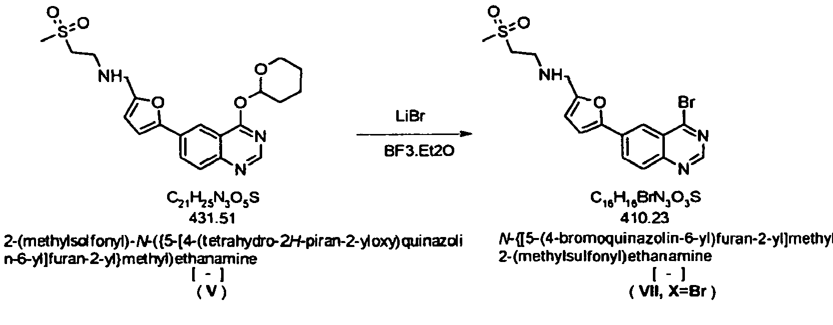

In a 4-neck glass flask provided with a stirrer 4.32 g of 2-(methylsulfonyl)-*N*-({5-[4-(tetrahydro-2*H*-pyran-2-yloxy)quinazoline-6-yl]furan-2-yl}methyl)ethanamine of formula (V) (10 mmoles), 0.87 g of LiBr (10 mmoles) and 10 mL of acetonitrile were introduced. Under a nitrogen atmosphere and maintaining the temperature at about 0°C there were added 1.56 g of freshly distilled BF₃.Et₂O (11 mmoles). The mixture was brought to ambient temperature and stirred for 24 hours. The mixture was thus concentrated to residue and the residue was recovered using 200 mL of ether. The solution was washed using a 10% sodium thiosulfate solution, then using water, then using brine. The organic phase was dried using anhydrous Na₂SO₄ then concentrated to residue. There are obtained 3.08 g of *N*-{[5-(4-bromoquinazoline-6-yl)furan-2-yl]methyl}-2-(methylsulfonyl)ethanamine of formula (VII, X=Br) with a molar yield equivalent to 75%.

Example 10 - Preparation of *N*-{[5-(4-iodoquinazoline-6-yl)furan-2-yl]methyl}-2-(methylsulfonyl)ethanamine of formula (VII, X=I) (variant)- exemplifying the invention.

### Synthesis scheme

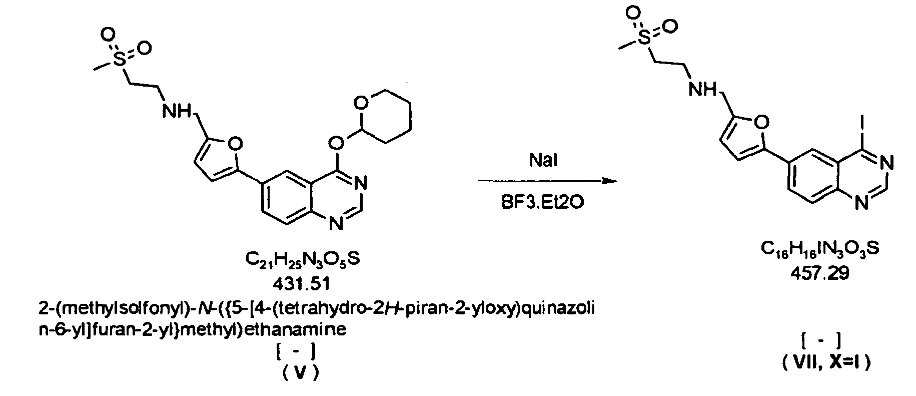

The process is repeated like in example 9 where 1.50 g of sodium iodide (NaI, 10 mmoles) are used instead of LiBr. There are obtained 3.2 g of *N*-{[5-(4-iodoquinazoline-6-yl)furan-2-yl]methyl}-2-(methylsulfonyl)ethanamine of formula (VII, X=I) with a molar yield equivalent to 70%.

## Claims

1. Process for the preparation of Lapatinib of formula (I) or a salt thereof: 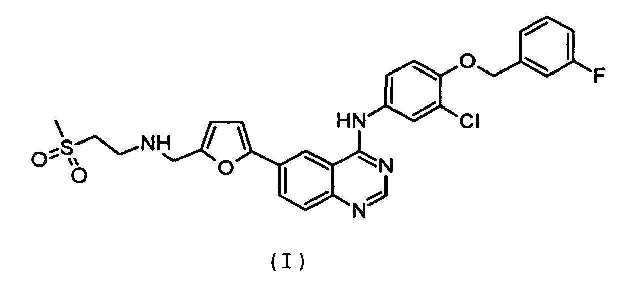 comprising the following steps:
(a) Reaction of 6-iodoquinazolin-4-ol of formula (II): 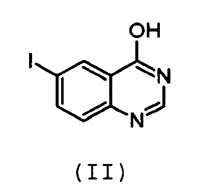 with the 3,4-dihydro-2*H*-pyran to provide 6-iodo-4-(tetrahydro-2*H*-pyran-2-yloxy)quinazoline of formula (III): 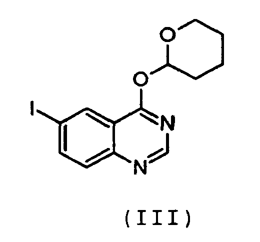
(b) Reaction of 6-iodo-4-(tetrahydro-2*H*-piran-2-yloxy)quinazoline of formula (III) with 2-formylfuran-5-boronic acid to provide 5-[4-(tetrahydro-2*H*-piran-2-yloxy)quinazolin-6-yl]furan-2-carbaldehyde of formula (IV): 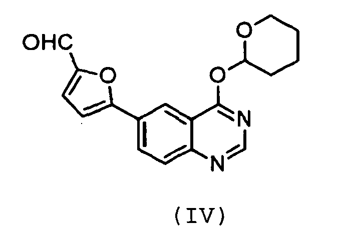
(c) Reaction of the intermediate of formula (IV) with the 2-(methylsulfonyl)ethanamine to provide 2-(methylsulfonyl)-*N*-{(5-[4-(tetrahydro-2*H*-piran-2-yloxy)quinazolin-6-yl]furan-2-yl}methyl)ethanamine of formula (V): 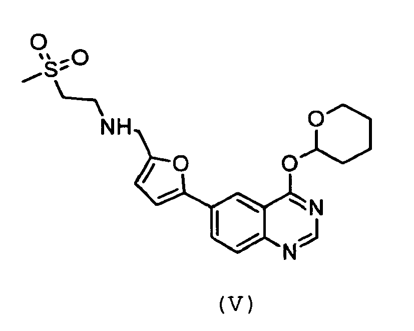
(d) Reaction of deprotection of the intermediate of formula (V) to provide 6-[5-({[2-(methylsulfonyl)ethyl]amino}methyl)furan-2-yl] quinazolin-4-ol of formula (VI): 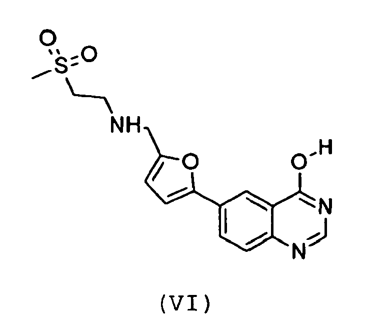
(e) Conversion of the intermediate of formula (VI) to provide *N*-{[5-(4-substituted-quinazolin-6-yl)furan-2-yl]methyl}-2-(methylsulfonyl) ethanamine of formula (VII): 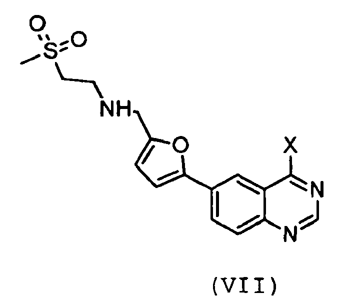 in which X is chosen in the group consisting in fluorine, chlorine, bromine, iodine, O-Ms (mesilate), O-Ts (tosilate), O-Tf (triflate);
(f) Reaction of the intermediate of formula (VII) with 3-chloro-4-[(3-fluorobenzyl)oxy]aniline of formula (VIII): 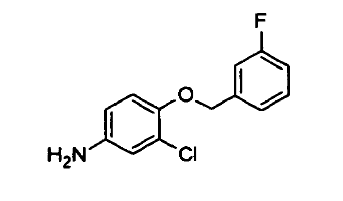 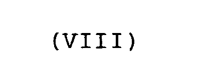 to provide Lapatinib of formula (I): 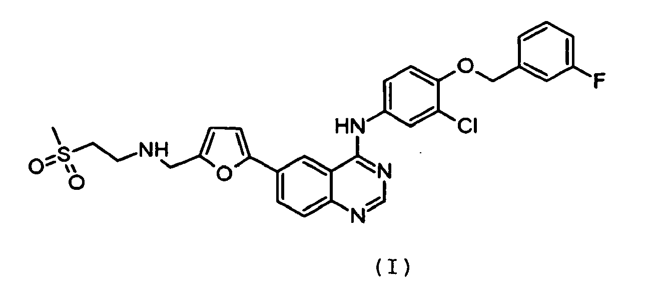
(g) Optionally, conversion of the Lapatinib of formula (I) in Lapatinib ditosilate monohydrate of formula (I-bis);
or,
(h) process in which the steps (d) and (e) are susbstituted by the reaction in which the intermediate 2-(methylsulfonyl)-*N*-({5-[4-(tetrahydro-2*H*-piran-2-yloxy)quinazolin-6-yl]furan-2-yl}methyl)ethanamine of formula (V) is directly converted in *N*-{[5-(4-bromoquinazolin-6-yl)furan-2-yl]methyl}-2-(methylsulfonil)ethanamine of formula (VII, X=Br): 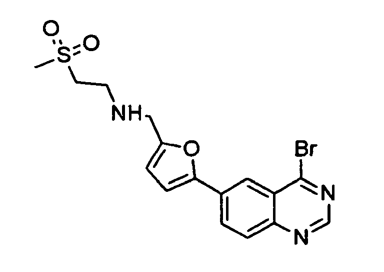 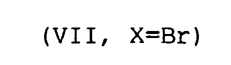
or in *N*-{[5-(4-iodoquinazolin-6-yl)furan-2-yl]methyl}-2-(methylsulfonyl)ethanamine of formula (VII, X=I): 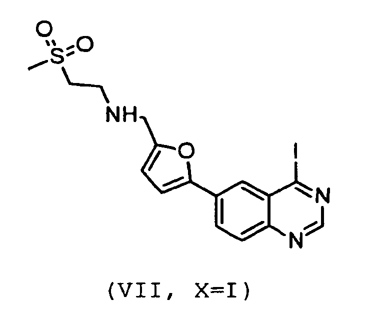 and the step (f) is performed with the intermediate of formula (VII, X=Br) or of formula (VII, X=I).

2. Process for the preparation of 2-(methylsulfonyl)-*N*-({5-[4-(tetrahydro-2H-piran-2-yloxy)quinazolin-6-yl]furan-2-yl}methyl)ethanamine of formula (V) or a salt thereof: 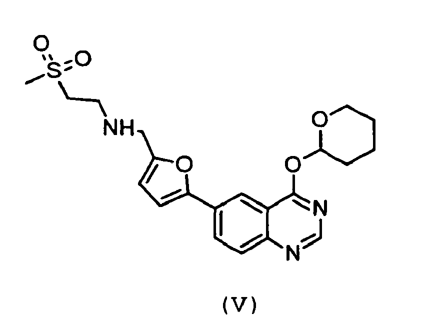 comprising the following steps:
(a) Reaction of 6-iodoquinazolin-4-ol of formula (II) : 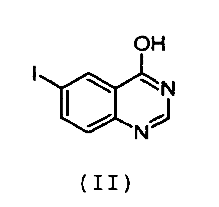 with 3,4-dihydro-2*H*-piran to provide 6-iodo-4-(tetrahydro-2*H*-piran-2-yloxy)quinazoline of formula (III): 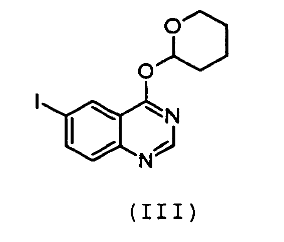
(b) Reaction of 6-iodo-4-(tetrahydro-2*H*-piran-2-yloxy)quinazoline of formula (III) with 2-formylfuran-5-boronic acid to provide 5-[4-(tetrahydro-2*H*-piran-2-yloxy)quinazolin-6-yl]furan-2-carbaldehyde of formula (IV): 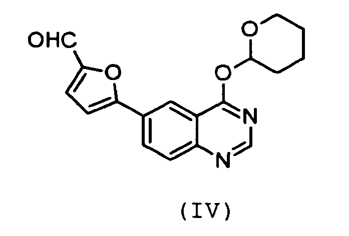
(c)Reaction of the intermediate of formula (IV) with 2-(methylsulfonyl)ethanamine.

3. Process according to any of claims from 1 to 2 in which the step (a) is performed in toluene.

4. Process according to any of claims from 1 to 3 in which the step (a) comprises the recrystallization of the product of formula (III) from ethyl acetate.

5. Process according to the claims from 1 to 2 in which the step (b) is performed in anhydrous conditions and in absence of oxygen.

6. Process according to the claims from 1 to 2 in which the step (b) is performed in presence of palladium tris dibenzylydeneacetone.

7. Process according to the claims from 1 to 2 in which the step (c) is performed in presence of sodium triacetoxyborohydride.

8. Process according to the claim 1 in which the step (d) is performed in an alcoholic solvent.

9. Process according to claim 1 in which the step (e) is performed in presence of phosphorus oxychloride.

10. Process according to the claim 1 in which the product of the step (e) is *N*-{[5-(4-Chloro-quinazolin-6-yl)furan-2-yl]methyl}-2-(methylsulfonyl)ethanamine of formula (VII, X=Cl):

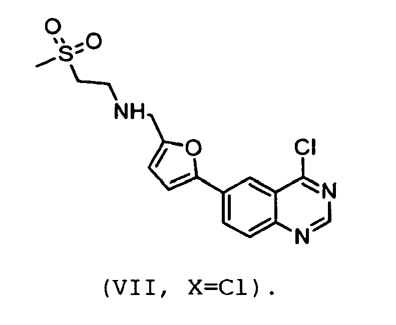

11. Process according to claim 1 in which the step (f) is performed in isopropanol.

12. Process according to the claim 1 in which the step (h) is performed by means of NaBr or NaI and BF3-Et20 or trimethylchlorosilane.

13. Compound selected in the group consisting of:
(a) 6-iodo-4-(tetrahydro-2*H*-piran-2-yloxy)quinazoline of formula (III),
(b) 5-[4-(tetrahydro-2*H*-piran-2-yloxy)quinazolin-6-yl]furan-2-carbaldehyde of formula (IV),
(c) 2-(methylsulfonyl)-*N*-{(5-[4-(tetrahydro-2H-piran-2-yloxy)quinazolin-6-yl]furan-2-yl}methyl) ethanamine of formula (V),
(d) 6-[5-({[2-(methylsulfonyl)ethyl]amino}methyl) furan-2-yl]quinazolin-4-ol of formula (VI),
(e) *N*-{[5-(4-substituted-quinazolin-6-yl)furan-2-yl]methyl}-2-(methylsulfonyl)ethanamine of formula (VII): 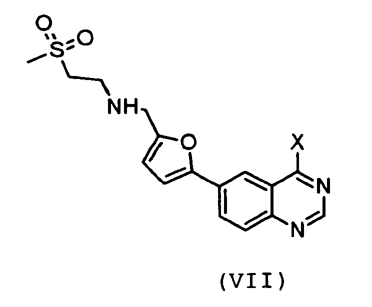 in which X is chosen in the group consisting of fluorine, chlorine, bromine, iodine, O-Ms (mesilate), O-Ts (tosilate), O-Tf (triflate); and salt thereof.

14. Use of the compounds according to the claim 13 for the preparation of Lapatinib of formula (I) and salts thereof.

## Patentansprüche

1. Verfahren zur Herstellung von Lapatinib der Formel (I) oder eines Salzes davon: 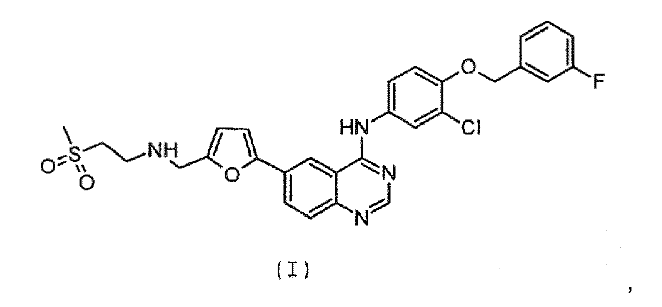 umfassend die folgenden Schritte:
(a) Umsetzen von 6-lod-chinazolin-4-ol der Formel (II) 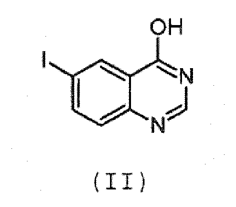 mit dem 3,4-Dihydro-2H-pyran, um 6-lod-4-(tetrahydro-2H-pyran-2-yloxy)chinazolin der Formel (III) bereitzustellen: 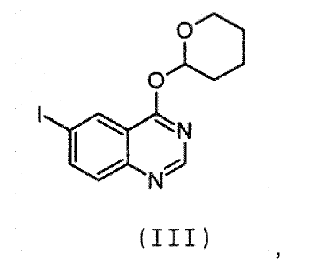
(b) Umsetzen von 6-lod-4-(tetrahydro-2H-pyran-2-yloxy)chinazolin der Formel (III) mit 2-Formylfuran-6-borsäure, um 5-[4-(tetrahydro-2H-pyran-2-yloxy)chinazolin-6-yl]furan-2-carbaldehyd der Formel (IV) bereitzustellen: 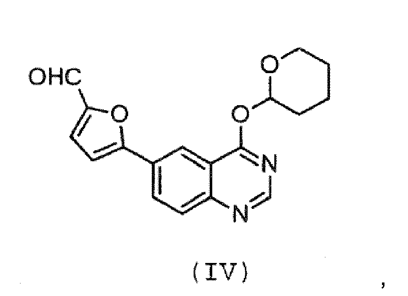
(c) Umsetzen des Zwischenprodukts der Formel (IV) mit 2-(Methylsulfonyl)ethanamin, um 2-(Methylsulfonyl)-N-({5-(4-(tetrahydro-2H-pyran-2-yloxy)chinazolin-6-yl]furan-2-yl}methyl)ethanamin der Formel (V) bereitzustellen: 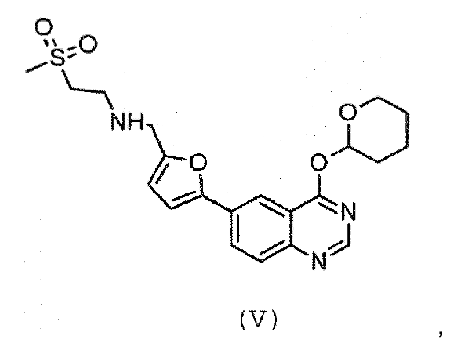
(d) Entschützungsreaktion des Zwischenprodukts der Formel (V), um 6-[5-({[2-(methylsulfonyl)ethyl]amino}methyl)furan-2-yl}chinazolin-4-ol der Formel (VI) bereitzustellen: 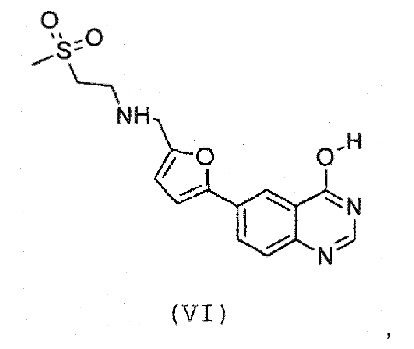
(e) Umsetzen des Zwischenprodukts der Formel (VI), um N-{[5-(4-substituiertes-Chinazolin-6-yl)furan-2-yl]methyl}-2-(methylsulfonyl]ethanamin der Formel (VII) herzustellen: 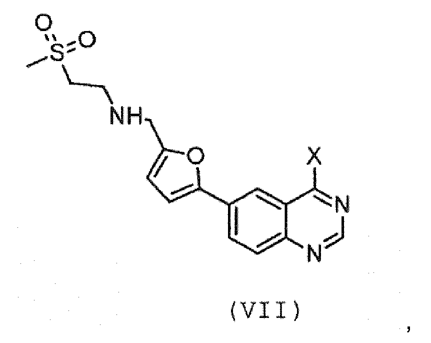 worin X ausgewählt wird aus der Gruppe, bestehend aus Fluor, Chlor, Brom, lod, O-Ms (Mesylat), O-Ts (Tosylat), O-Tf (Triflat);
(f) Umsetzen des Zwischenprodukts der Formel (VII) mit 3-Chlor-4-[(3-fluorbenzyl)oxy]anilin der Formel (VIII) 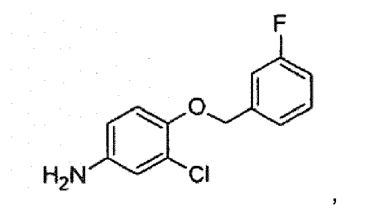 um Lapatinib der Formel (I) bereitzustellen: 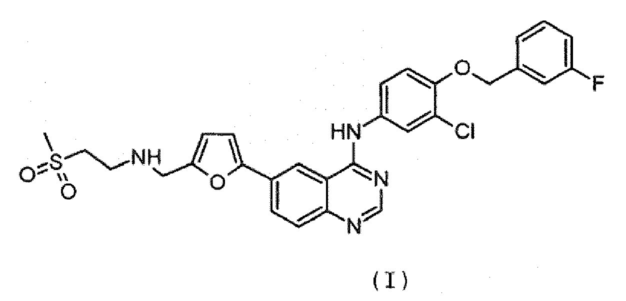
(g) optional Umsetzen des Lapatinibs der Formel (I) in Lapatinibditosylat-monohydrat der Formel (I-bis); oder
(h) Verfahren, worin die Schritte (d) und (e) ersetzt werden durch die Reaktion, worin das Zwischenprodukt 2-(Methylsulfonyl)-N-({5-(4-(tetrahydro-2H-pyran-2-yloxy)chinazolin-6-yl]furan-2-yl}methyl)ethanamin der Formel (V) direkt in N-{[5-(4-Bromchinazolin-6-yl}furan-2-yl]methyl}-2-(methylsulfonyl)ethanamin der Formel (VII, X=Br) übergeführt wird: 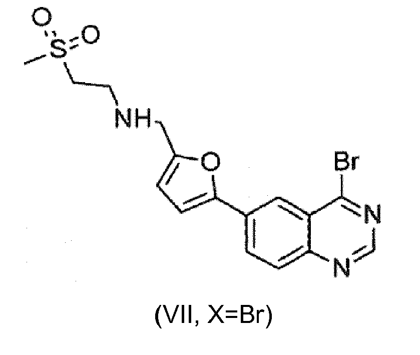 oder in N-{[5-(4-lodchinazolin-6-yl}furan-2-yl]methyl}-2-(methylsulfonyl)ethanamin der Formel (VII, X=I) 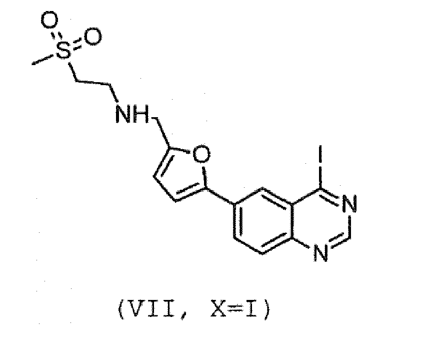 und der Schritt (f) mit dem Zwischenprodukt der Formel (VII, X=Br) oder der Formel (VII, X=I) durchgeführt wird.

2. Verfahren zur Herstellung von 2-(Methylsulfonyl)-N-({5-(4-(tetrahydro-2H-pyran-2-yloxy)chinazolin-6-yl]furan-2-yl}methyl)ethanamin der Formel (V) oder eines Salzes davon: 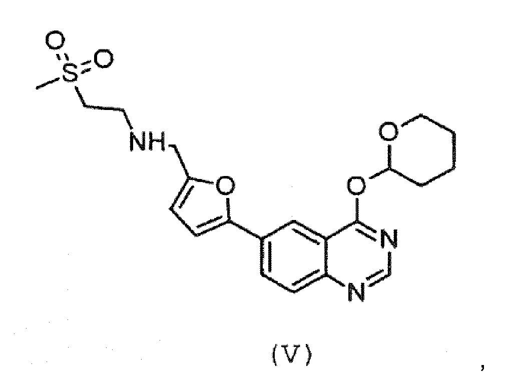 umfassend die folgenden Schritte:
(a) Umsetzen von 6-lodchinazolin-4-ol der Formel (II) 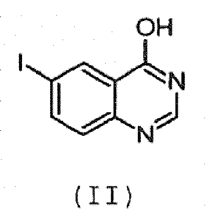 mit 3,4-Dihydro-2-H-pyran, um 6-lod-4-(Tetrahydro-2-H-pyran-2-yloxy)chinazolin der Formel (III) bereitzustellen: 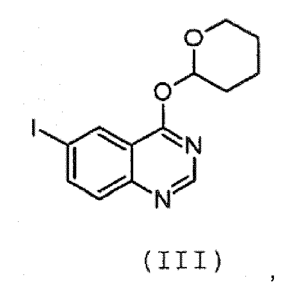
(b) Umsetzen von 6-lod-4-(tetrahydro-2-H-pyran-2-yloxy)chinazolin der Formel (III) mit 2-Formylfuran-5-borsäure, um 5-[4-(Tetrahydro-2H-pyran-2yl-oxy)chinazolin-6-yl]furan-2-carbaldehyd der Formel (IV) bereitzustellen: 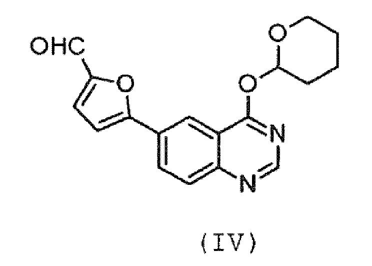
(c) Umsetzen des Zwischenprodukts der Formel (IV) mit 2-(Methylsulfonyl)ethanamin.

3. Verfahren nach einem der Ansprüche 1 bis 2, worin der Schritt (a) in Toluol durchgeführt wird.

4. Verfahren nach einem der Ansprüche 1 bis 3, worin Schritt (a) die Umkristallisation des Produkts der Formel (III) aus Ethylacetat umfasst.

5. Verfahren nach einem der Ansprüche 1 bis 2, worin Schritt (b) unter wasserfreien Bedingungen und in der Abwesenheit von Sauerstoff durchgeführt wird.

6. Verfahren nach einem der Ansprüche 1 bis 2, worin Schritt (b) in der Gegenwart von Palladium-tris-dibenzylidenaceton durchgeführt wird.

7. Verfahren nach einem der Ansprüche 1 bis 2, worin Schritt (c) in der Gegenwart von Natriumtriacetoxyborhydrid durchgeführt wird.

8. Verfahren nach Anspruch 1, worin Schritt (d) in der Gegenwart eines Alkohollösungsmittels durchgeführt wird.

9. Verfahren nach Anspruch 1, worin Schritt (e) in der Gegenwart von Phosphoroxychlorid durchgeführt wird.

10. Verfahren nach Anspruch 1, worin des Produkt von Schritt (e) N-{[5-(4-Chlorchinazolin-6-yl}furan-2-yl]methyl}2-(methylsulfonyl)ethanamin der Formel (VII, X=Cl) ist:

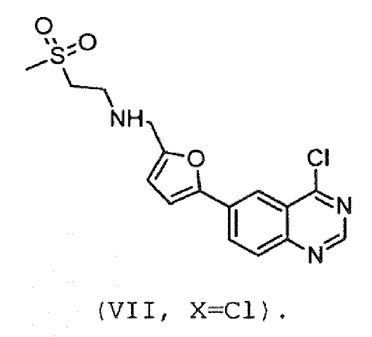

11. Verfahren nach Anspruch 1, worin Schritt (f) in Isopropanol durchgeführt wird.

12. Verfahren nach Anspruch 1, worin Schritt (h) mithilfe von NaBr oder Nal und BF₃-Et₂O oder Trimethylchlorsilan durchgeführt wird.

13. Verbindung, ausgewählt aus der Gruppe, bestehend aus:
(a) 6-lod-4-(tetrahydro-2H-pyran-2-yloxy)chinazolin der Formel (III),
(b) 5-[4-(Tetrahydro-2H-pyran-2-yloxy)chinazolin-6-yl]furan-2-carbaldehyd der Formel (IV),
(c) 2-(Methylsulfonyl)-N-({5-(4-(tetrahydro-2H-pyran-2-yloxy)chinazolin-6-yl]furan-2-yl}methyl)ethanamin der Formel (V)
(d) 6-[5-({[2-(Methylsulfonyl)ethyl]amino}methyl)furan-2-yl}chinazolin-4-ol der Formel (VI) bereitzustellen,
(e) N-{[5-(4-substituiertes-Chinazolin-6-yl)furan-2-yl]methyl}-2-(methylsulfonyl]ethanamin der Formel (VII): 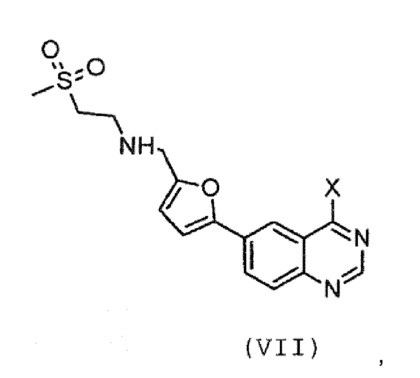 worin X ausgewählt ist aus der Gruppe, bestehend aus Fluor, Chlor, Brom, lod, O-Ms (Mesylat), O-Ts (Tosylat), O-Tf (Triflat);
und Salze davon.

14. Verwendung der Verbindungen nach Anspruch 13 zur Herstellung von Lapatinib der Formel (I) und Salzen davon.

## Revendications

1. Procédé pour la préparation de lapatinib de la formule (I) ou d'un sel de celui-ci : 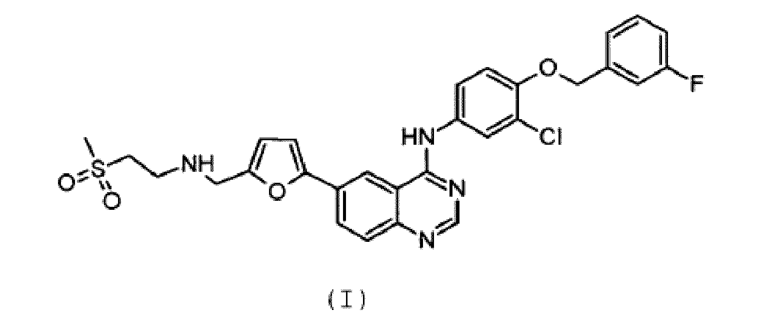 comprenant les étapes suivantes :
(a) de réaction de 6-iodoquinazolin-4-ol de la formule (II) : 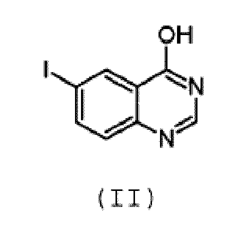 avec le 3,4-dihydro-2*H*-pyrane pour fournir de la 6-iodo-4-(tétrahydro-2*H*-pyran-2-yloxy)quinazoline de la formule (III) : 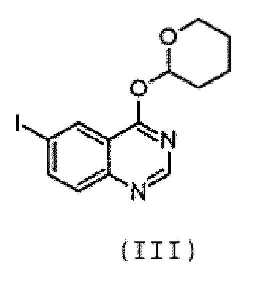
(b) de réaction de 6-iodo-4-(tétrahydro-2*H*-pyran-2-yloxy)quinazoline de la formule (III) avec de l'acide 2-formylfuran-5-boronique pour fournir du 5-[4(tétrahydro-2*H*-pyran-2-yloxy)quinazolin-6-yl]furan-2-carbaldéhyde de la formule (IV) : 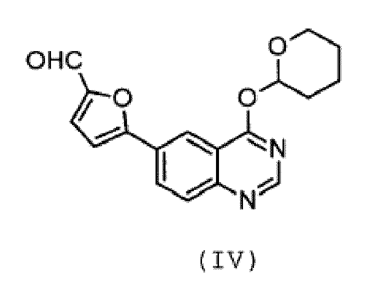
(c) de réaction de l'intermédiaire de la formule (IV) avec la 2-(méthyl-sulfonyl)éthanamine pour fournir de la 2-(méthylsulfonyl)-*N*-({5-[4-(tétrahydro-2*H*-pyran-2-yloxy)quinazolin-6-yl]furan-2-yl}méthyl)-éthanamine de la formule (V) : 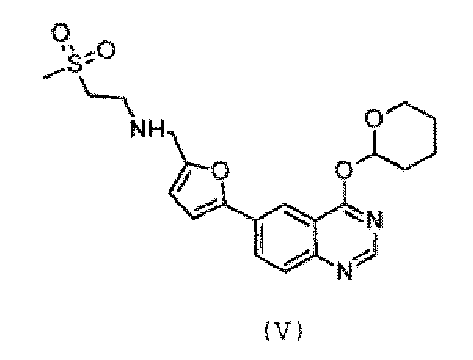
(d) de réaction de déprotection de l'intermédiaire de la formule (V) pour fournir du 6-[5-({[2-(méthylsulfonyl)éthyl]amino}méthyl)furan-2-yl]-quinazolin-4-ol de la formule (VI) : 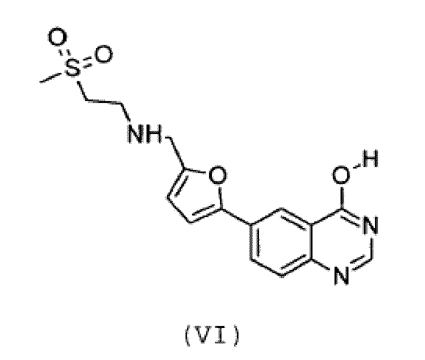
(e) de conversion de l'intermédiaire de la formule (VI) pour fournir de la *N*-{[5-(quinazolin-6-yle 4-substitué)furan-2-yl]méthyl}-2-(méthylsulfonyl)éthanamine de la formule (VII) : 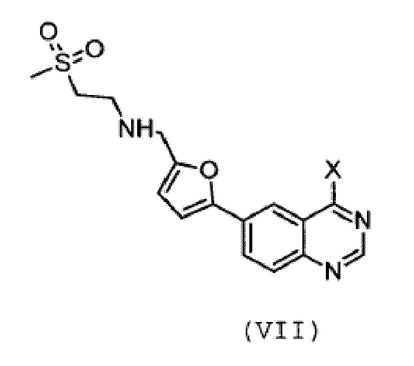 dans laquelle X est choisi dans le groupe constitué par le fluor, le chrome, le brome, l'iode, l'O-Ms (mésilate), l'O-Ts (tosilate), l'O-Tf (triflate) ;
(f) de réaction de l'intermédiaire de la formule (VII) avec de la 3-chloro-4-[(3-fluorobenzyl)oxy]aniline de la formule (VIII) : 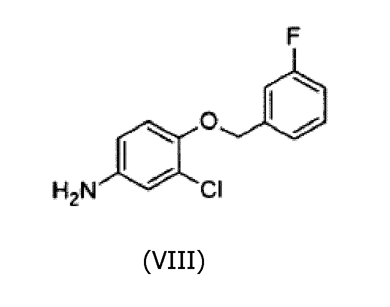 pour fournir du lapatinib de la formule (I) : 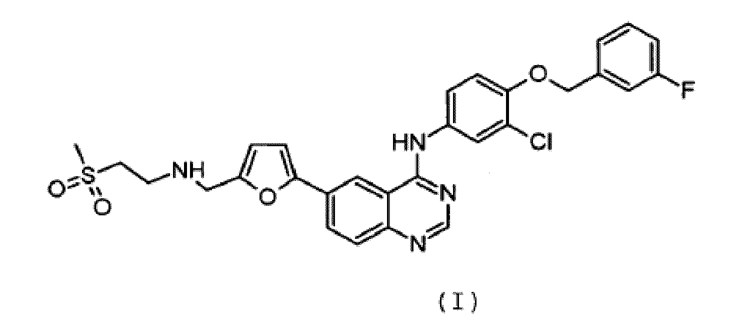
(g) facultativement, de conversion du lapatinib de la formule (I) en monohydrate de ditosilate de lapatinib de la formule (I-bis) ;
ou,
(h) procédé dans lequel les étapes (d) et (e) sont substituées par la réaction dans laquelle l'intermédiaire de 2-(méthylsulfonyl)-*N*-({5-[4-(tétrahydro-2*H*-pyran-2-yloxy)quinazolin-6-yl]furan-2-yl}méthyl)-éthanamine de la formule (V) est directement converti en *N*-{[5-(4-bromoquinazolin-6-yl)furan-2-yl]méthyl}-2-(méthylsulfonyl)éthanamine de la formule (VII, X=Br) : 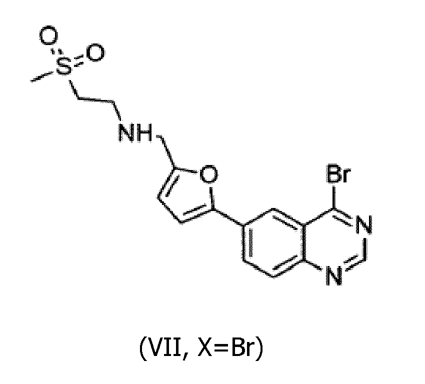 ou e n *N*-{[5-(4-iodoquinazolin-6-yl)furan-2-yl]méthyl}-2-(méthyl-sulfonyl)éthanamine de la formule (VII, X=I) : 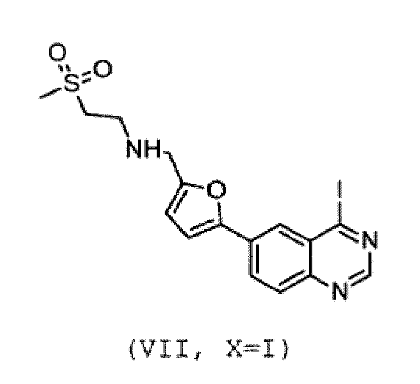 et l'étape (f) est réalisée avec l'intermédiaire de la formule (VII, X=Br) ou de la formule (VII, X=I).

2. Procédé pour la préparation de 2-(méthylsulfonyl)-*N*-({5-[4-(tétrahydro-2*H*-pyran-2-yloxy)quinazolin-6-yl]furan-2-yl}méthyl)éthanamine de la formule (V) ou d'un sel de celle-ci : 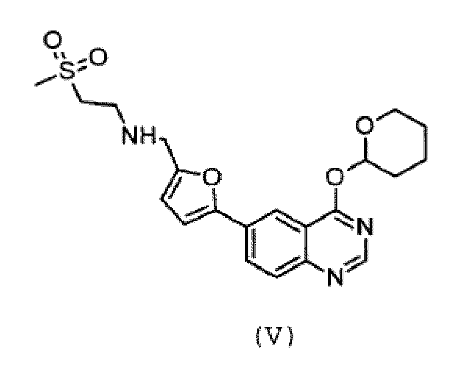 comprenant les étapes suivantes :
(a) de réaction de 6-iodoquinazolin-4-ol de la formule (II) : 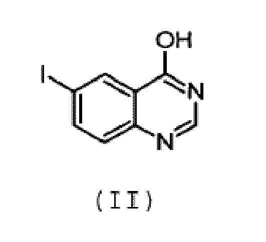 avec du 3,4-dihydro-2*H*-pyrane pour fournir de la 6-iodo-4-(tétrahydro-2*H*-pyran-2-yloxy)quinazoline de la formule (III) : 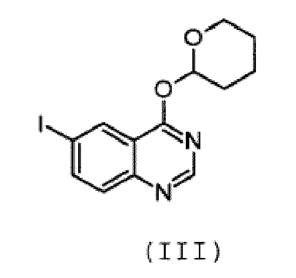
(b) de réaction de la 6-iodo-4-(tétrahydro-2*H*-pyran-2-yloxy)quinazoline de la formule (III) avec de l'acide 2-formylfuran-5-boronique pour fournir du 5-[4-(tétrahydro-2*H*-pyran-2-yloxy)quinazolin-6-yl]furan-2-carbaldéhyde de la formule (IV) : 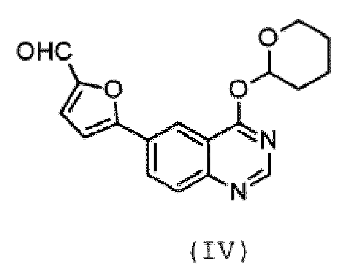
(c) de réaction de l'intermédiaire de la formule (IV) avec de la 2-(méthylsulfonyl)éthanamine.

3. Procédé selon l'une quelconque des revendications 1 à 2, dans lequel l'étape (a) est réalisée dans du toluène.

4. Procédé selon l'une quelconque des revendications 1 à 3, dans lequel l'étape (a) comprend la recristallisation du produit de la formule (III) à partir d'acétate d'éthyle.

5. Procédé selon les revendications 1 à 2, dans lequel l'étape (b) est réalisée dans des conditions anhydres et en l'absence d'oxygène.

6. Procédé selon les revendications 1 à 2, dans lequel l'étape (b) est réalisée en présence de tris(dibenzylidèneacétone)palladium.

7. Procédé selon les revendications 1 à 2, dans lequel l'étape (c) est réalisée en présence de triacétoxyborohydrure de sodium.

8. Procédé selon la revendication 1, dans lequel l'étape (d) est réalisée dans un solvant alcoolique.

9. Procédé selon la revendication 1, dans lequel l'étape (e) est réalisée en présence d'oxychlorure de phosphore.

10. Procédé selon la revendication 1, dans lequel le produit de l'étape (e) est la *N*-{[5-(4-chloro-quinazolin-6-yl)furan-2-yl]méthyl}-2-(méthylsulfonyl)éthanamine de la formule (VII, X=Cl) :

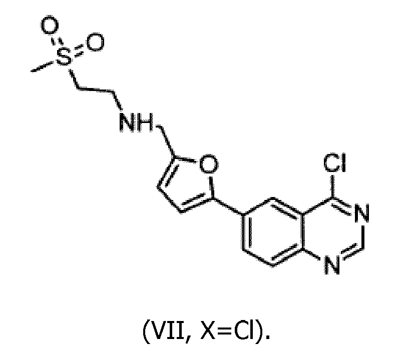

11. Procédé selon la revendication 1, dans lequel l'étape (f) est réalisée dans de l'isopropanol.

12. Procédé selon la revendication 1, dans lequel l'étape (h) est réalisée au moyen de NaBr ou de NaI et de BF3-Et20 ou de triméthyl-chlorosilane.

13. Composé choisi dans le groupe constitué par :
(a) la 6-iodo-4-(tétrahydro-2*H*-pyran-2-yloxy)quinazoline de la formule (III),
(b) le 5-[4-(tétrahydro-2*H*-pyran-2-yloxy)quinazolin-6-yl]furan-2-carbaldéhyde de la formule (IV),
(c) 1 a 2-(méthylsulfonyl)-*N*-({5-[4-(tétrahydro-2*H*-pyran-2-yloxy)quinazolin-6-yl]furan-2-yl}méthyl)éthanamine de la formule (V),
(d) le 6-[5-({[2-(méthylsulfonyl)éthyl]amino}méthyl)furan-2-yl] quinazolin-4-ol de la formule (VI),
(e) la *N*-{[5-(quinazolin-6-yle 4-substitué)furan-2-yl]méthyl}-2-(méthylsulfonyl)éthanamine de la formule (VII) : 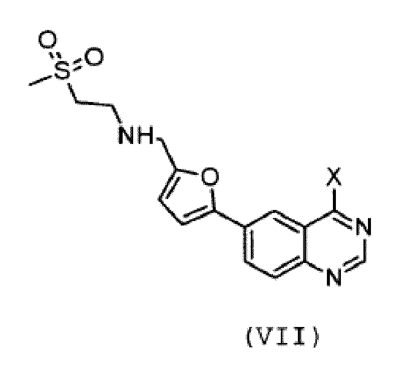 dans laquelle X est choisi dans le groupe constitué par le fluor, le chlore, le brome, l'iode, l'O-Ms (mésilate), l'O-Ts (tosilate), l'O-Tf (triflate) ; et sel de celui-ci.

14. Utilisation des composés selon la revendication 13 pour la préparation de lapatinib de la formule (I) et de sels de celui-ci.
